## Europäisches Patentamt

## European Patent Office

## Office européen des brevets

(19)

(11) Publication number: **0 036 138**
**B2**

(12)

# NEW EUROPEAN PATENT SPECIFICATION

(45) Date of publication of the new patent specification: 25.10.89

(51) Int. Cl.⁴: **A 61 K 7/48,** A 61 K 9/06

(21) Application number: 81101583.3

(22) Date of filling: 05.03.81

(54) Composition of matter for topical application comprising a bio-affecting agent and N,N-diethyl-m-toluamide.

(30) Priority: 06.03.80 US 127881
06.03.80 US 127883

(43) Date of publication of application:
23.09.81 Bulletin 81/38

(45) Publication of the grant of the patent:
28.08.85 Bulletin 85/35

(45) Mention of the opposition decision:
25.10.89 Bulletin 89/43

(84) Designated Contracting States:
AT BE CH DE FR GB IT LI LU NL

(56) References cited:
FR-A-2 368 889
US-A-3 452 139
US-A-3 711 602
US-A-3 821 363
US-A-4 006 218

THE MERCK INDEX, 9th edition, 1976, Rahway,
Merck & Co. page 1080, abstract no. 9093 "Salicylic
Acid"
Remington's Pharmacuetical Science, 15th Ed.
1975, p. 1206, 1093, 1094
P. Albert, "Selective Toxiaty", 5th Ed. 1979,
Chapmand Hall, P. 60-61
Ariens, "Drug Design", Vol. II, 1973 Acad. Press. p.
172-173
M.S. BALSAM and E. SAGARIN Cosmetics,
Science and Technology, Soc. Ed., vol. 2, (1972),
Wiley Interscience, pp. 128-134

(73) Proprietor: **MERCK & CO. INC., 126, East Lincoln
Avenue P.O. Box 2000, Rahway New Jersey
07065- 0900 (US)**

(72) Inventor: **Windheuser, John J., Am Buchenwald 9,
D-8133 Feldafing (DE)**
Inventor: **Haslam, John L., R.R. 2 Box 259- D,
Lawrence Kansas 66044 (US)**
Inventor: **Caldwell, Larry J., 917 Maine, Lawrence
Kansas 66044 (US)**

(74) Representative: **Abitz, Walter, Dr.- Ing., Abitz,
Morf, Gritschneder P.O. Box 86 01 09, D-8000
München 86 (DE)**

(56) References cited: (continuation)
HARRY'S Cosmeticology, vol. 1, Leonard Hill
Books, 1973, pp. 428-437
Maison G. Navarre, "The Chemistry and
Manufacture of Cosmetics, 2nd ed., vol. 4,
Continental Press (1975), pp. 1125-1143

The file contains technical information submitted
after the application was filed and not included in
this specification

EP 0 036 138 B2

## Description

The invention provides novel compositions for topical application comprising at least one bio-affecting agent selected from the group as mentioned below together with N,N-diethyl-m-toluamide (DEET) and, if desired, a non-toxic topical carrier. Formulation of DEET with the bio-affective agent has been found to greatly enhance delivery of the bio-affecting agent through the protective outer layer of the skin and into the underlying tissues or into the general circulation.

As used herein, the term "bio-affecting agent" refers to any chemical substance within the group as mentioned below or formulation thereof which beneficially affects the mammaiian body. Typically, the bio-affecting agent herein can be a "dermally effective dermatological agent" or a "systemically effective therapeutic agent". The term "dermally effective dermatological agent" as used herein refers to those chemical substances which when applied to mammalian skin exert a beneficial topical effect, which can be of a cosmetic nature (e.g. by protecting the skin against external factors or by improving appearance) or of a therapeutic nature (e.g., by attenuating the severity of a dermal disease). The term "systemically effective therapeutic agent" as used herein refers to those chemical substances which when administered by various routes such as intravenous infusion, intramuscular injection, oral, rectal or buccal routes, enter the general circulation and exhibit a therapeutic effect. The expressions "dermally effective dermatological agent" and "systemically effective therapeutic agent" are not intended to be mutually exclusive, however, it being recognized that a number of bio-affecting agents are indeed effective both dermally and systemically.

## Background of the Prior Art

N,N-Diethyl-m-toluamide (DEET) is a well-known chemical compound which has long been used as an insect repellant. Various synthesis for the preparation thereof too are well-known to the art. Compare The Merck Index, (1976), page 3125, Ninth Edition, and the references cited therein. See also United States Patent Nos. 2 932 665 and 4 064 268.

Moreover, it is know from US-A-3 452 139 to use DEET in lotions containing isopropanol or in conventional shampoos, hair rinses and hair tonics (see US-A-3 452 139, column 2, lines 22 - 26 and 55 - 66). US-A-3 821 863 describes sunscreen preparations which may contain DEET.

The skin of humans and other warm-blooded animals provides an excellent barrier to the penetration of exogenous chemical substances. The outer layer of the epidermis, called the stratum corneum, offers the maximum resistance to penetration, whereas the lower layers are relatively permeable. For proper treatment of dermal conditions, it is important that the active agent penetrate the stratum corneum where it is retained. From this reservoir in the outer layer, the bio-affecting agent is slowly released and penetrates the underlying areas where it exhibits its therapeutic or cosmetic effect. When dermatological agents such as sunscreens, which protect the underlying tissues from external factors (ultraviolet rays) are used, maximal retention in the stratum corneum is essential. On the other hand, the relative permeability of the layers of the epidermis below the stratum corneum can also allow access to the systemic circulation, indeed, it is necessary for a therapeutic agent to penetrate the stratum corneum in order to provide systemic therapeusis from the transdermal route.

It is well-known that the application of various therapeutic and cosmetic agents to the skin is useful for the treatment of a number of dermal conditions, e.g., hydrocortisone for pruritus and erythema in a topic dermatitis, erythromycin or tetracyclines for acne, 5-iodo-2'-deoxyuridine for herpes simplex, 5-fluorouracil for psoriasis and skin cancer, hydroquinone for lightening skin color and p-aminobenzoic acid for blocking the harmful rays of the sun.

It is also well-known that a number of therapeutically active agents, such as $\beta$-blockers, antihypertensives, antiarrhythmics, antianginal agents, vasodilators, antiemetics, antibacterials, antifungals, corticosteroids, progestins, estrogens, androgens and antiinflammatories, when administered to warm-blooded animals by a number of various routes such as by intravenous infusion, intramuscular injection, oral, rectal or buccal routes, enter the general circulation and produce the appropriate systemic therapeutic effect. It is also known that the aforementioned methods of administration have certain disadvantages. For example, the intravenous and intramuscular routes are not only painful for the patient, but also must be performed by a trained individual. Buccal and rectal administration often produce discomfort and annoyances for the patient. Oral administration, although generally acceptable for the patient, often does not deliver the majority of the therapeutic agent to systemic circulation. This diminished drug delivery is usually attributed to poor absorption from the gastrointestinal tract and/or to degradation of the agent by the acidic medium of the stomach, by the enzymes in the gastrointestinal tract and surrounding tissue or by the rapid metabolozing enzymes of the liver through which the drug must pass before it enters the systemic circulation. For example, drugs such as anti-bacterials, narcotic analgesics, $\beta$-blockers and others require relatively high doses when given orally due to the remarkable liver metabolism encountered. Effective delivery of such drugs through the skin would require much lower doses because the so-called "first pass" metabolism would be avoided.

Recognizing the fact that the outer layer of the skin, the epidermis, protects the area under the skin from the penetration of foreign chemicals, many investigators have turned to various enhancing agents, e.g., dimethylsulfoxide, dimethylformamide, methyldecylsulfoxide (United States patent No. 3 527 864) and dimethylacetamide (United States patent No. 3 472 931) in order to overcome the aforementioned problems and to deliver topically active agents more efficiently through the skin, as well as to enhance the absorption of systemically active therapeutic agents through the skin and into the general circulation. Dimethylsulfoxide, which is superior to both dimethylformamide and dimethylacetamide, has been shown to enhance the absorption through the skin of hydrocortisone and testosterone (Robert J. Feldmann and Howard I. Maibach, Proceedings of the Joint Conference on Cosmetic Science (1968), pages 189 - 203). Thus, the addition of dimethylsulfoxide to formulations of therapeutically active agents enhances the penetration of said agents through the skin and into the general circulation, thereby overcoming most of the aforementioned problems encountered by other routes of administration. Unfortunately, the use of dimethylsulfoxide is not without problems, for in addition to causing foul taste and body odor, it causes burning and erythema on the skin, activates latent virus infections within cells, reduces the relucency of the lens cortex and produces teratogenicity and tissue necrosis in animals. Compare Martindale, The Extra Pharmacopoeia, pages 1461 - 1463, Twenty-seventh Edition (1977), and the references cited therein.

Thus, there exists a clear and present need for a novel agent to enhance the absorption through the skin of bio-affecting agents which is devoid of the disadvantages and drawbacks that to date have characterized the prior art enhancing agents.

**Objects and Summary of the Invention**

Accordingly, a major object of the present invention is the provision of a novel agent for enhancing the skin-permeation of certain bio-affecting agents. More particularly, it is a major object of the present invention to provide a novel agent which will enhance the dermal absorption of certain dermatological (i.e. therapeutic or cosmetic) agents and which will enhance the delivery through the skin and into the general circulation of certain systemically active therapeutic agents.

Another object of the invention is to provide such an enhancing agent which is devoid of toxic side effects.

Yet another object of the invention is to provide novel compositions utilizing the aforesaid novel enhancing agent for topical application and novel methods of enhancing the skin penetration of bio-affecting chemicals utilizing said enhancing agent.

In accord with the foregoing, the invention provides novel compositions of matter for topical application comprising a biologically effective amount of at least one bio-affecting agent selected from antibacterial, antifungal, antiviral, corticosteroidal, steroidal antiinflammatory, non-steroidal antiinflammatory, antipsoriatic, antidermatitis, antipruritic, antiwart, keratolytic, local anesthetic, antierythema, antimetabolite, progestational, estrogenic, androgenic, emollient, skin lightening, antihistaminic, antiacne, antiperspirant, β-blocker, antihypertensive, antianginal, antiarrhythmic, vasodilator, antiemetic, muscle relaxant or antiasthma agents and a skin permeation enhancing amount of N,N-diethyl-m-toluamide (DEET), further comprising, if desired, a non-toxic topical carrier with the exception of combinations of N,N-diethyl-m-toluamide with isopropanol or with shampoos, hair tonics or hair rinses. The term "a biologically effective amount" means an amount sufficient to produce the desired biological effect. Thus, when the bio-affecting agent is a dermatological agent, it is utilized in a dermally effective amount, i.e., in an amount sufficient to evoke the desired dermal effect (which may be cosmetic or therapeutic in nature). On the other hand, when the bio-affecting agent is a systemically active therapeutic agent and introduction of the agent into the general circulation is desired, then the agent is employed in a systemically effective amount, i.e., in an amount sufficient to produce the desired systemic response. DEET is employed in the present compositions in an amount sufficient to enhance skin permeation of the bio-affecting agent.

**Detailed Description of the Invention**

The present invention provides compositions for surprisingly and greatly increasing the rate of penetration through the skin of various bio-affecting agents. The amount of dermatological agents absorbed into the underlying tissues of the skin and the amount of systemically effective therapeutic agents absorbed into the general circulation can be dramatically increased utilizing the compositions and method of the invention.

Many substances provide beneficial effects when applied topically to the skin, for example, for the purpose of treating surface or subsurface diseases or for creating skin conditions which protect the skin from external factors. Such dermatological agents, which can be made more useful by enhancing their penetration through the protective layer of the skin in accord with the present invention, are exemplified by, but not limited to, the following classes of substances:

(a) Antibacterial, antifungal, antiacne and antiviral agents. These substances, which have been shown to have increased percutaneous absorption when used in the present process, are illustrated by lincomycin; clindamycin; tetracycline, oxytetracycline, chlorotetracycline, and other tetracycline-type antibiotics; erythromycin; 2-thiopyridine N-oxide; halogen compounds, particularly iodine and iodine compounds such as iodine-PVP complex and diiodohydroxyquin; penicillins, such as penicillin G and penicillin V; cephalosporins, i.e. any of the many new forms of these β-lactam antibiotics such as cephalexin; any of the sulfonamide class of antibacterials; hexachlorophene; chlorhexidine; chloroamine compounds; benzoylperoxide; streptomycin or any other members of the class of aminoglycoside antibiotics; nitrofurantoin; nystatin, amphotericin B; 5-iodo-2-deoxyuridine; griseofulvin; thiabenzadole; and gramicidin. When the level of said agents in the skin is greatly increased, the host has an improved ability to combat dermal infections such as boils, infected cuts or incisions, acne, herpes sores and ringworm. Experimental data has shown that DEET formulations of these agents enhance the delivery of the agents into the skin.

(b) Antimetabolites, for example, 5-fluorouracil, 6-mercaptopurine, mycophenolic acid and methotrexate, which have utility in the treatment of skin cancers and psoriasis. These agents have shown increased dermal penetration and increased efficacy when formulated with DEET.

(c) Anticholinergic agents, which are effective for the inhibition of axillary sweating and for the control of prickly heat. The antiperspirant activity of agents such as methatropine nitrate, propantheline bromide, scopolamine, methscopolamine bromide, and the new class of soft antiperspirants, quaternary acyloxymethyl ammonium salts described, for example, by Bodor et al., J. Med. Chem. 23, (1980) 474 and also in United Kingdom Specification No. 2 010 270, published 27 June 1979 is greatly enhanced when formulated with DEET.

(d) Steroidal antiinflammatory agents, such as hydrocortisone, hydrocortisone 17-valerate, hydrocortisone 17-butyrate, hydrocortisone 21-acetate, betamethasone valerate, triamcinolone acetonide, fluocinonide, desonide, fluocinolone acetonide, dexamethasone, dexamethasone 21-phosphate, prednisolone, prednisolone 21-phosphate, and haloprednone; as well as non-steroidal antiinflammatory agents, such as indomethacin, naproxen, fenoprofen, ibuprofen, alcolfenac, phenylbutazone, sulindac, desoxysulindac, diflunisal, aspirin and mefenamic acid. These agents are effective for treating inflammatory disorders of the skin and the underlying tissues, and the rate and extent of their penetration is greatly enhanced by formulation with DEET. Further examples of steroidal antiinflammatory agents for use in the instant compositions include cortisone acetate, hydrocortisone cyclopentylpropionate, cortodoxone, flucetonide, fludrocortisone acetate, flurandrenolone acetonide, medrysone, amcinafal, amcinafide, betamethasone, betamethasone benzoate, chloroprednisone acetate, clocortolone acetate, descinolone acetonide, desoximetasone, dichlorisone acetate, difluprednate, flucloronide, flumethasone, flumethasone pivalate, flunisolide acetate, fluocortolone, fluorometholone, fluperolone acetate, fluprednisolone, fluprednisolone valerate, meprednisone, methyl prednisolone, paramethasone acetate, prednisolamate, prednisone, prednival, triamcinolone, triamcinolone hexacetonide, cortivazol, formocortal and nivazol. Additional non-steroidal antiinflammatory agents which can be formulated in combination with DEET include salicylamide, salicyclic acid, flufenisal, salsalate, triethanolamine salicylate, aminopyrine, antipyrine, oxyphenbutazone, apazone, cintazone, flufenamic acid, clonixeril, clonixin, meclofenamic acid, flunixin, colchicine, demecolcine, allopurinol, oxypurinol, benzydamine hydrochloride, dimefadane, indoxole, intrazole, mimbane hydrochloride, paranylene hydrochloride, tetrydamine, benzindopyrine hydrochloride, fluprofen, ibufenac, ketoprofen, naproxol, fenbufen, cinchophen, diflumidone sodium, fenamole, flutiazin, metazamide, letimide hydrochloride, nexeridine hydrochloride, octazamide, molinazole, neocinchophen, nimazole, proxazole citrate, tesicam, tesimide, tolmetin, tramadol and triflumidate.

(e) Local anesthetics, such as dibucaine and lidocaine. Such agents are poorly absorbed through the skin but when formulated with DEET show enhanced anesthetic properties.

(f) Sex hormones, i.e. the estrogens, androgens and progestins, particularly the natural sex hormones estradiol, testosterone and progesterone, which are useful for a variety of cosmetic purposes such as stimulation of scalp hair growth and use in beauty preparations. DEET added to these preparations enhances the penetration of the hormones and increases their retention.

(g) Antihistamines, such as cyproheptadine hydrochloride (Periactin). These too can be adventageously formulated in combination with DEET.

(h) Miscellaneous dermatological agents, e.g. skin lightening agents such as hydroquinone, keratolytics and agents for treating psoriasis, dermatitis, pruritus and erythema, and emollients.

A wide variety of therapeutic agents provide beneficial effects when absorbed into the systemic circulation. Formulations of such systemically effective therapeutic agents in combination with DEET greatly enhances their rate of penetration through the skin and the amount absorbed into the systemic circulation, and thus makes it possible to achieve a systemic effect through topical application of the drug. There is a significant advantage to the topical delivery of systemically effective therapeutic agents in cases where the drug is not absorbed well orally, produces gastric problems, or even if well absorbed, is rapidly metabolized in the liver immediately after absorption (the "first pass" effect). In such cases, by using topical delivery, a systemic response can be elicited at a lower dosage than required orally. At the same time, topical delivery avoids the disadvantages inherent in the intravenous route of administration, which would otherwise be necessary to achieve effective blood levels at reasonable dosage amounts. Such systemically effective therapeutic agents, which can be formulated in combination with DEET, are exemplified by, but not limited to, the following classes of substances:

(a) β-Blockers, such as propranolol, bupranolol, metoprolol, nadolol, satalol, alprenolol, oxprenolol, carteolol, labetalol, atenolol, pindolol, timolol and timolol maleate. Because these antiarrhythmic agents are subject to extensive liver metabolism, elevated doses are required orally for clinical efficacy. Thus, the formulations of DEET with these agents are especially advantageous.

(b) Antibacterial, antifungal and antiviral agents. These substances, which have been shown to have increased percutaneous absorption when used in accord with the present invention, are exemplified by lincomycin; clindamycin; tetracycline, oxytetracycline, chlorotetracycline and other tetracycline-type antibiotics; erythromycin; 2-thiopyridine N-oxide; halogen compounds, especially iodine compounds; penicillins, such as penicillin G and penicillin V; cephalosporins, i.e., any of the many new forms of these β-lactam antibiotics such as cefalexin and cefoxytin; any of the sulfonamide class of antibacterials; streptomycin or any other members of the class of aminoglycoside antibiotics; nitrofurantoin; nystatin; amphotericin B; 5-iodo-2-deoxyuridine; N-formimidoyl thienamycin monohydrate; 1-ethyl-6-fluoro-1,4-dihydro-4-oxo-7-(1-piperazinyl)-3-quinoline carboxylic acid; phosphonomycin; novabiocin; cycloserine; cephamycins, particularly cephamycin C; and griseofulvin. Experimental data show the DEET formulations of these agents enhance their delivery through the skin.

(c) Steroidal antiinflammatory agents, i.e., corticosteroids, such as hydrocortisone, hydrocortisone 17-valerate, hydrocortisone 17-butyrate, hydrocortisone 21-acetate, betamethasone valerate, triamcinolone acetonide, fluocinonide, desonide, fluocinolone acetonide, dexamethasone, dexamethasone 21-phosphate, prednisolone, prednisolone 21-phosphate, haloprednone, cortisone acetate, hydrocortisone cyclopentylpropionate, cortodoxone, flucetonide, fludrocortisone acetate, flurandrenolone acetonide, medrysone, amcinafal, amcinafide, betamethasone, betamethasone benzoate, chloroprednisone acetate, clocortolone acetate, descinolone acetonide, desoximetasone, dichlorisone acetate, difluprednate, flucloronide, flumethasone, flumethasone pivalate, flunisolide acetate, fluocortolone, fluorometholone, fluperolone acetate, fluprednisolone, fluprednisolone valerate, meprednisone, methyl prednisolone, paramethasone acetate, prednisolamate, prednisone, prednival, triamcinolone, triamcinolone hexacetonide, cortivazol, formocortal and nivazol. These compounds, which can be of great value systemically in inflammation, are better absorbed through the skin when formulated with DEET.

(d) Non-steroidal antiinflammatory agents, such as indomethacin, naproxen, fenoprofen, ibuprofen, alcolfenac, phenylbutazone, mefenamic acid, sulindac, desoxysulindac, diflunisal, aspirin, salicylamide, salicylic acid, flufenisal, salsalate, triethanolamine salicylate, aminopyrine, antipyrine, oxyphenbutazone, apazone, cintazone, flufenamic acid, clonixeril, clonixin, meclofenamic acid, flunixin, colchicine, demecolcine, allopurinol, oxypurinol, benzydamine hydrochloride, dimefadane, indoxole, intrazole, mimbane hydrochloride, paranylene hydrochloride, tetrydamine, bezindopyrine hydrochloride, fluprofen, ibufenac, ketoprofen, naproxol, fenbufen, cinchophen, diflumidone sodium fenamole, flutiazin, metazamide, letimide hydrochloride, nexeridine hydrochloride, octazamide, molinazole neocinchophen, nimazole, proxazole citrate, tesicam, tesimide, tolmetin, tramadol and triflumidate. These compounds are effective for treating inflammatory disorders of the skin and the underlying tissues. The rate and extent of penetration of these agents is greatly enhanced by the formulation with DEET.

(e) Antihypertensives, such as clonidine and α-methyldopa, and antiangina and vasodilator agents such as nitroglycerin, erythritol tetranitrate, isosorbide dinitrate, mannitol hexanitrate, pentaerythrityl tetranitrate, papaverine and dipyridamole. Such agents show enhanced absorption through the skin when formulated with DEET.

(f) Sex hormones, i.e. the estrogens, androgens and progestins, especially the natural sex hormones estradiol, testosterone and progesterone. These agents show very poor bioavailability by the oral route, but are well absorbed through the skin when formulated with DEET.

(g) Muscle relaxants, for example cyclobenzaprine hydrochloride and diazepam. These can be advantageously formulated in combination with DEET.

(h) Antiasthma drugs, such as cromoglycic acid and its prodrugs [described, for example, in International Journal of Pharmaceutics, 7, (1980) 63 - 75]. Because of its short half-life, cromoglycic acid is an especially desirable candidate for formulation with DEET according to the present invention.

(i) Antiemetics, e.g. pipamazine, chlorpromazine, and dimenhydrinate. These can also be formulated with DEET in accord with the present invention.

The effectiveness of DEET as a penetration enhancer is illustrated by comparison of formulations of a number of representative bio-affecting agents with DEET vis-à-vis commercial formulations of those agents, or formulations of those agents with common adjuvants. The comparison made generally consisted of measuring the relative penetration through hairless mouse skin of the various formulations. In every case, the delivery observed with the DEET formulations was superior. In addition, the effectiveness of a DEET formulation of a representative systemically active drug was demonstrated in vivo using beagle dogs. Further, the dermal effectiveness of DEET formulations of dermatological agents was demonstrated in vivo by studying the vasoconstriction and blanching of the skin as induced by cortical steroids. When a commercial formulations of hydrocortisone 17-butyrate was compared to a DEET formulation, the latter shown comparable blanching at 1/10 of the dose of the commercial formulation.

Skin penetration was determined using an in vitro diffusion cell procedure. The diffusion cells were obtained from Kercso Engineering Consultants, 3248 Kipling Street, Palo Alto, California 94306. The plexiglass diffusion cells consisted of a lower chamber with a side arm to allow sampling of the receptor phase, and a teflon lid. A teflon®-coated stirring bar provided efficient mixing. The hairless mice (Jackson Labs) were sacrificed using cervical dislocation and the whole dorsal skin removed. The skin was gently stretched over the lower opening of the teflon lid and secured with a neoprene rubber gasket. The lid was then placed firmly on the lower chamber and secured with three screws. The opening in the lid left exposed an area of 8.0 cm$^2$ (3.2 cm in diameter) on the epidermis side through which penetration was measured. The receptor fluid was 45 mL of buffer consisting of 1.5/10$^{-1}$M NaCl, 5.0 x 10$^{-4}$M NaH$_2$PO$_4$, 2.0 x 10$^{-4}$M Na$_2$HPO$_4$ and 200 ppm gentamycin sulfate adjusted to pH 7.2 with sodium hydroxide or hydrochloric acid. Air bubbles were carefully removed from the dermal surface of the skin by tipping the cell. In most cases 100 mg of formulation, solution or suspension containing the drug substance to be tested was applied evenly over the mouse skin. The cell was placed in a thermostated chamber maintained at $32 \pm 1°C$ and the reservoir stirred by a magnetic stirrer at 2.5 Hz. After 24 hours a sample of the receptor fluid was withdrawn by a pipet through the side arm and emptied into a test tube, capped and frozen. The concentration of applied drug in each diffusion cell sample was measured using high pressure liquid chromatography (HPLC). The results reported for each experiment are the average values from three replicate diffusion cells. Chromatography was performed on a high capability chromatograph using assay conditions specific for measurement of the target compounds in each example. Conditions are described in each example.

In addition to the diffusion cell technique to measure percutaneous absorption, hydrocortisone 17-butyrate was also tested on humans. Test formulations were applied to test areas (2 cm x 2 cm) on the lower back of healthy male and female human volunteer subjects. Each formulation was applied to four or more test areas per subject and the results averaged. All test areas were exposed to air (non occluded) for 3.5 hours after application, at which time any remaining formulation was wiped off with ethanol. Blanching was evaluated at 5, 6, 7 and 8 hours post application. The blanching at each test area was visually appraised on a scale of 0-4 where 0 = no blanching, 1 = barely discernible blanching, 4 = maximum blanching. Individual values for each formulation are averaged (N = 8 - 10) and this value becomes the basis for different comparisons. In the Examples which follow, percents are by weight unless otherwise specified.

**Example 1**

The _in vitro_ diffusion cell method described above was used to compare the penetration of a 1 % hydrocortisone solution in DEET with a commercial product (Hytone® cream containing 1 % hydrocortisone). The DEET solution was prepared by dissolving 30 mg of hydrocortisone in 2.97 g of DEET. A 100 mg sample of the solution or a 100 mg sample of the Hytone® cream was applied to the mouse skin in the diffusion cell experiment. Reservoir samples were assayed for hydrocortisone by HPLC using a μBondapak® C18 column with detection at 254 nm. The mobile phase was 35 % by volume acetonitrile/ 65 % by volume water.

**Table I**

| Hydrocortisone in | 24 hour permeation percent |
|---|---|
| Hytone® cream[a] | $1.6 \pm 0.1$ |
| DEET | $35 \pm 14$ |

[a]Dermik Laboratories Inc., Ft. Washington, PA 19034

**Example II**

The _in vitro_ diffusion cell method described previously was used to compare the permeation of hydrocortisone 21-acetate from a solution in DEET and a commercial product (Carmol® 1 % hydrocortisone acetate cream). The DEET solution was prepared by dissolving 30 mg of hydrocortisone 21-acetate in 2.97 g of DEET. A 100 mg sample of this solution or a 100 mg sample of Carmol® cream was applied to the mouse skin in the diffusion cell experiment. Samples of the reservoir fluid were assayed for hydrocortisone 21-acetate by HPLC using an RP-8 column with detection at 254 nm. The mobile phase was 20 % by volume acetonitrile/20 % by volume tetrahydrofuran/60 % by volume water.

**Table II**

| Hydrocortisone 21-acetate in | 24 hour permeation percent |
|---|---|
| Carmol® cream[a] | $0.67 \pm 0.12$ |
| DEET | $28 \pm 4$ |

[a]Ingram Pharmaceutical Co., San Francisco, CA 94111.

**Example III**

The in vitro diffusion cell procedure described previously was used to compare the permeation of hydrocortisone 17-butyrate from a solution in DEET with a commercial product (Locoid® cream at 0.1 % hydrocortisone 17-butyrate). The 0.1 % solution of hydrocortisone 17-butyrate was prepared by dissolving 30 mg of hydrocortisone 17-butyrate in 3 g of DEET. A 100 mg sample of this solution or a 100 mg sample of Locoid® cream was applied to the skin. Samples of the reservoir fluid were assayed for hydrocortisone 17-butyrate by HPLC using an RP-8 column with detection at 254 nm. The mobile phase was 40 % by volume tetrahydrofuran/60 % by volume water.

**Table III**

| Hydrocortisone 17-butyrate in | 24 hour permeation percent |
|---|---|
| Locoid® cream[a] | $4.7 \pm 0.6$ |
| DEET | $63 \pm 7$ |

[a]Torii Pharmaceutical Co., Ltd., Tokyo, Japan

**Example IV**

The in vitro diffusion cell procedure previously described was used to compare the permeation of hydrocortisone 17-valerate from a solution in DEET with a commercial product (Westcort® cream, 0.2 % hydrocortisone 17-valerate). A 0.2 % solution of hydrocortisone 17-valerate was prepared by dissolving 6 mg of the steroid in 3 g of DEET. A 100 mg sample of this solution or a 100 mg sample of Westcort® cream was applied to the skin. Samples of the reservoir fluid were assayed for hydrocortisone 17-valerate by HPLC using an RP-8 column with detection at 254 nm. The mobile phase was 30 % by volume acetonitrile/20 % by volume tetrahydrofuran/50 % by volume water.

**Table IV**

| Hydrocortisone 17-valerate in | 24 hour permeation percent |
|---|---|
| Westcort® cream[a] | $6.1 \pm 0.8$ |
| DEET | $41 \pm 10$ |

[a]Westwood Pharmaceutical, Inc., Buffalo, NY 14213.

**Example V**

The in vitro diffusion cell procedure previously described was used to compare the permeation of a solution of erythromycin in DEET with a suspension in petrolatum. The 1 % solution of erythromycin base was prepared by dissolving 30 mg of erythromycin base in 2.97 g of DEET. The 1 % suspension in petrolatum was prepared by mixing 30 mg of erythromycin base with melted petrolatum till cool. A 100 mg sample of the solution or of the suspension was applied to the skin. Samples of the reservoir fluid were assayed for erythromycin by HPLC using a µBondapak® C18 column with detection at 215 nm. The mobile phase was 2 mM $NH_4H_2PO_4$ in 30 % by volume acetonitrile/70 % by volume water.

**Table V**

| Erythromycin in | 24 hour permeation percent |
|---|---|
| Petrolatum | not detectable |
| DEET | $83 \pm 6$ |

**Example VI**

The in vitro diffusion cell procedure previously described was used to compare the permeation of tetracycline from a suspension in DEET with a commercial product (Topicycline® solution). The Topicycline® (2.2 mg of the tetracycline hydrochloride per mL as active ingredient) was prepared as described on the package insert. A suspension containing the same 2.2 mg amount of tetracycline hydrochloride per mL was prepared in DEET using the same mixture of solid ingredients (tetracycline hydrochloride, 4-epitetracycline hydrochloride and sodium bisulfite) as in Topicycline. Samples of 100 mg were applied to the skin. Samples of the reservoir fluid were assayed for tetracycline by HPLC with detection at 254 nm. The mobile phase was 1 mM $Na_2EDTA$ and 2 mM $H_3PO_4$ in 28 % by volume acetonitrile/72 % by volume water.

**Table VI**

| Tetracycline in | 24 hour permeation percent |
|---|---|
| Topicycline®a | 0.05 |
| DEET | 6 |

aProcter & Gamble, Cincinnati, OH 45202

### Example VII

The _in vitro_ diffusion cell procedure described previously was used to compare the permeation of dibucaine from a solution in DEET with a commarcial product (Nupercainal® cream, 0.5 % dibucaine). A solution of dibucaine was prepared by dissolving 15 mg of dibucaine in 2.98 g of DEET. A 100 mg sample of the solution or a 100 mg sample of the cream was applied to the skin. Samples of the reservoir fluid were assayed for dibucaine by HPLC using a µBondapak® RP-Cn column with detection at 254 nm. The mobile phase was 2 mM $NH_4H_2PO_4$ in 40 % by volume acetonitrile/60 % by volume water.

**Table VII**

| Dibucaine in | 24 hour permeation percent |
|---|---|
| Nupercainal® creama | $15 \pm 1$ |
| DEET | $83 \pm 11$ |

aCIBA Pharmaceutical Co., Summit, NJ 07901

### Examples VIII

The _in vitro_ diffusion cell procedure described previously was used to compare the permeation of indomethacin in a DEET solution with a suspension in petrolatum. A 1 % solution of indomethacin was prepared by dissolving 30 mg of indomethacin in 2.97 g of DEET. A 1 % suspension was prepared by mixing 30 mg of indomethacin with 2.97 g of melted petrolatum until cool. A 100 mg sample of the solution or a 100 mg sample of the suspension was applied to the skin. Samples of reservoir fluid were assayed for indomethacin by HPLC using a µBondapak® RP-Cn column with detection at 254 nm. The mobile was 2 mM $NH_4H_2PO_4$ in 40 % by volume acetonitrile/60 % by volume water.

**Table VIII**

| Indomethacin in | 24 hour permeation percent |
|---|---|
| Petrolatum | $1 \pm 0.4$ |
| DEET | $38 \pm 16$ |

### Example IX

The _in vitro_ diffusion cell procedure described previously was used to compare the permeation of ibuprofen from a solution in DEET with a suspension in petrolatum. A 1 % solution of ibuprofen was prepared by dissolving 30 mg of ibuprofen in 2.97 g of DEET. A 1 % suspension of ibuprofen was prepared by mixing 30 mg of ibuprofen with 2.97 g of melted petrolatum until cool. Samples of reservoir fluid were assayed for ibuprofen by HPLC using a µBondapak® RP-Cn column with detection at 205 nm. The mobile phase was 2 mM $NH_4H_2PO_4$ in 35 % by volume acetonitrile/65 % by volume water.

**Table IX**

| Ibuprofen in | 24 hour permeation percent |
|---|---|
| Petrolatum | $64 \pm 7$ |
| DEET | $102 \pm 4$ |

**Examples X**

The in vitro diffusion cell procedure described previously was used to measure the dermal diffusion of quaternary ammonium salts useful as antiperspirants. Solutions of d,l-α-cyclopentylphenylacetoxymethyl-1-methylpyrrolidinium chloride dissolved in ethanol or 10 % DEET in ethanol (0.031 M, 250 μL) were applied to the epidermal surface (8.04 cm$^2$) of albino, hairless mouse skin in a diffusion cell. Samples of the receptor fluid (isotonic $6.7 \times 10^{-3}$M sodium phosphate pH 6.0, 32°C) were removed at timed intervals and the concentration of the quaternary salt was determined by HPLC analysis using a Partisil® SCX column eluted with $4 \times 10^{-2}$M potassium phosphate buffer pH 3.5 in 60 % by volume methanol/40 % by volume water. The peaks were detected at 254 nm.

**Table X**
Diffusion of quaternary salt through skin

| Solvent | % Diffusion at 24 hours |
|---|---|
| Ethanol | 3.0 |
| 10 % DEET/Ethanol | 67.3 |

**Example XI**

A 1 % solution of hydrocortisone 17-butyrate was prepared in a 1 : 1 mixture of isopropyl myristate and DEET (30 mg of hydrocortisone 17-butyrate in 2.97 g of isopropyl myristate/DEET mixture). For the blanching test, 1 μl of the above solution and 50 μl of Locoid® cream were applied to the skin. The results are shown in Table XI.

**Table XI**

| Formulation of hydrocortisone 17-butyrate | Amount applied | Blanching response at various times, hours | | | |
|---|---|---|---|---|---|
| | | 5 | 6 | 7 | 8 |
| Locoid® cream[a] 0.1 % | 50 μl | 3.9 ± 0.9 | 3.0 ± 0.9 | 3.3 ± 0.8 | 3.5 ± 0.5 |
| DEET/IPM (1 : 1) 1 % | 1 μl | 2.3 ± 1.2 | 3.0 ± 0.9 | 2.8 ± 1.0 | 2.7 ± 0.8 |

[a]Torii Pharmaceutical Co., Ltd., Tokyo, Japan

A similar blanching response was obtained with the DEET/IPM at 1/5 the applied dose which indicates a five fold better delivery of hydrocortisone 17-butyrate in DEET/IPM over Locoid® cream.

**Example XII**

The underarm antiperspirant efficacy of d,l-α-cyclopentylacetoxymethyl-1-methylpyrrolidinium dodecyl sulfate was measured in ethanol and DEET. The quaternary salt (17 mg) in ethanol or DEET was applied to the right armpit of healthy female human subjects and allowed to dry. The percent inhibition of perspiration was determined 5 hours after three daily applications. The two subjects were subjected to heat stress (40°C) and their perspiration output compared to the perspiration of the control left armpit.

**Table XII**
Axillary antiperspirant activity in ethanol or DEET
% Inhibition after

| Subject | Solvent | 3 daily applications |
|---------|---------|----------------------|
| 1 | Ethanol | 21 |
|   | DEET | 34 |
| 2 | Ethanol | 29 |
|   | DEET | 37 |

## Examples XIII

The in vitro diffusion method described hereinabove was used to determine the dermal penetration of bupranolol in DEET compared to other commonly used penetration enhancers. In each case the solvent was saturated with excess drug at 25 and 100 μl of the suspension was applied to the epidermal side of the mouse skin. Reservoir samples were assayed for bupranolol by HPLC using a μBondapak® RP-CN column with detection at 205 nm. The mobile phase was 60 % by volume acetonitrile/40 % by volume water, 2 mM in $NH_4H_2PO_4$.

### Table XIII

| Bupranolol in | 24 hour penetration |
|---------------|----------------------|
| DEET | 16 mg |
| Propylene Glycol | 16 mg |

## Examples XIV

In order to test the in vivo effect of bupranolol penetration, 200 mg of bupranolol was suspended in 1 ml of DEET and the suspension was absorbed onto a 6 $cm^2$ woven fiberglass patch backed with aluminum foil. The patch was applied to the clipped back of a beagle dog which was challenged at periodic intervals with 50 μl of isoproterenol (1 : 5000). The ratio of heart rate increase as compared to control was taken as a measure of the percent of beta blockade. The percent beta blockade increased continuously and was 45 % at 5 hours when the experiment was terminated.

## Examples XV

The in vitro diffusion cell procedure described previously was used to compare the permeation of griseofulvin in a DEET solution with a suspension of petrolatum. A 0.5 % solution of griseofulvin was prepared by dissolving 15 mg of griseofulvin in 2.985 g of DEET. A 0.5 % suspension was prepared by mixing 15 mg of griseofulvin with 2.985 g of melted petrolatum until cool. A 100 mg sample of the solution or a 100 mg sample of the suspension was applied to the skin. Samples of reservoir fluid were assayed for griseofulvin by HPLC using a μBondapak® RP-CN column with detection at 295 nm. The mobile phase was 2 mM $NH_4H_2PO_4$ in 25 % by volume acetonitrile/75 % by volume water.

### Table XIV

| Griseofulvin in | 24 hour permeation percent |
|-----------------|----------------------------|
| Petrolatum | 0.4 |
| DEET | 29.0 |

## Example XVI

The in vitro diffusion cell procedure described previously was used to compare the permeation of triethanolamine salicylate from a solution in DEET with a commercial product (Asper® lotion, 10 % triethanolamine salicylate). A solution of triethanolamine salicylate was prepared by dissolving 1 g of triethanolamine salicylate in 9 g of DEET. A 100 mg sample of the solution or a 100 mg sample of the commercial lotion was applied to the skin. Samples of the reservoir fluid were assayed for triethanolamine

10

salicylate by HPLC using an RP-8 column with detection at 254 nm. The mobile phases was 10 mM $H_3PO_4$ in 60 % by volume methanol/40 % by volume water.

**Table XV**

| Triethanolamine salicylate in | 24 hour permeation percent |
| --- | --- |
| Asper® lotion[a] | 16 |
| DEET | 89 |

[a]Thompson Medical Co. Inc., New York, N.Y. 10022

**Example XVII**

The _in vitro_ diffusion cell procedure described previously was used to compare the permeation of mycophenolic acid in a DEET solution with a suspension in petrolatum. A 1 % solution of mycophenolic acid was prepared by dissolving 30 mg of mycophenolic acid in 2.97 g of DEET. A 1 % suspension was prepared by mixing 30 mg of mycophenolic acid with 2.97 g of melted petrolatum until cool. A 100 mg sample of the solution or a 100 mg sample of the suspension was applied to the skin. Samples of reservoir fluid were assayed for mycophenolic acid by HPLC using a μBondapak® RP-CN column with detection at 295 nm. The mobile phase was 2 mM $NH_4H_2PO_4$ in 10 % by volume acetonitrile/10 % by volume tetrahydrofuran/80 % by volume water.

**Table XVI**

| Mycophenolic acid in | 24 hour permeation percent |
| --- | --- |
| Petrolatum | Not detectable |
| DEET | 42 |

**Example XVIII**

The _in vitro_ diffusion cell procedure described previously was used to compare the permeation of idoxuridine (also known as 5-iodo-2-deoxyuridine) from a solution in DEET with a commercial product (Stoxil® solution, 0.1 % idoxuridine). A 0.1 % solution of idoxuridine was prepared by dissolving 30 mg of idoxuridine in 3 g of DEET. A 100 mg sample of that solution or a 100 mg sample of Stoxil® solution was applied to the skin. Samples of the reservoir fluid were assayed for idoxuridine by HPLC using an RP-8 column with detection at 254 nm. The mobile phase was 1 mM $H_3PO_4$ in 20 % by volume methanol/80 % by volume water.

**Table XVII**

| Idoxuridine in | 24 hour permeation percent |
| --- | --- |
| Stoxil® solution[b] | 8 |
| DEET | 77 |

[b]Smith Kline and French Laboratories, Philadelphia, PA 19101

**Example XIX**

The _in vitro_ diffusion cell procedure described previously was used to compare the permeation of dexamethasone from a suspension in DEET/petrolatum with a commercial product (Decaderm®, 0.1 % dexamethasone). A 0.1 % suspension of dexamethasone was prepared by warming a mixture of 5 g of DEET and 95 g of petrolatum until fluid (about 50°C), then adding 0.1 g of dexamethasone and mixing until cool. A 100 mg sample of the suspension in DEET/petrolatum or a 100 mg sample of the commercial preparation was applied to the skin. Samples of the reservoir fluid were assayed for dexamethasone by HPLC using a μBondapak® RP-CN column with detection at 254 nm. The mobile phase was 25 % by volume tetrahydrofuran /75 % by volume water.

**Table XVIII**

| Dexamethasone in | 6 hour permeation percent |
|---|---|
| Decaderm® topicalc | 8 |
| DEET/Petrolatum | 14 |

cMerck Sharp & Dohme, West Point, PA 19486

## Example XX

A 0.1 % suspension of dexamethasone in DEET/petrolatum was prepared as described in Example XIX. For the blanching test, samples of the above suspension and of Decaderm® topical were applied to the skin. The results are tabulated below.

**Table XIX**

| Formulation of dexamethasone | Amount applied | Blanching response at various times, hours | | | |
|---|---|---|---|---|---|
| | | 5 | 6 | 7 | 8 |
| Decaderm®, 0.1 %c | 50 mg | 1.8 ± 0.5 | 1.3 ± 1.0 | 1.3 ± 1.0 | 1.8 ± 0.5 |
| DEET/Petrolatum (5 : 95), 0.1 % | 50 mg | 3.8 ± 0.5 | 3.0 ± 0.8 | 2.8 ± 0.5 | 2.8 ± 0.5 |

cMerck Sharp & Dohme, West Point, PA 19486.

## Example XXI

The *in vitro* diffusion cell procedure described previously was used to compare the permeation of the following formulations of indomethacin: (a) 1 % indomethacin in petrolatum, prepared as described in Example VIII;

(b) 1 % indomethacin in DEET, prepared as described in Example VIII;

(c) 1 % indomethacin in 1 % DEET/petrolatum, prepared by warming a mixture of 1 g of DEET and 99 g of petrolatum until fluid (about 50°C), then adding 1 g of indomethacin and mixing until cool;

(d) 1 % indomethacin in 5 % DEET/petrolatum, prepared as in (c) above, but using 5 g of DEET and 95 g of petrolatum;

(e) 1 % indomethacin in 10 % DEET/petrolatum, prepared as in (c) above, but using 10 g of DEET and 90 g of petrolatum;

(f) 1 % indomethacin in an oil/water cream formulated as follows:

| Ingredient | % by weight |
|---|---|
| isopropyl myristate | 2 % |
| DEET | 5 % |
| petrolatum | 20 % |
| mineral oil | 20 % |
| sorbitan monostearate (Arlacel® 60) | 8 % |
| polysorbate 60 (Tween® 60) | 1 % |
| indomethacin | 1 % |
| water | 43 % |

The drug and all of the other ingredients except water were combined and heated to 70°C, with stirring, until homogeneous, then poured into water, which was also at 70°C, with rapid agitation. The mixture was stirred, as it was allowed to cool, until a creamy consistency was obtained;

(g) 1 % indomethacin in an oil/water cream formulated as follows:

| Ingredient | % by weight |
|---|---|
| isopropyl myristate | 2 % |
| DEET | 5 % |

| petrolatum | 20 % |
| mineral oil | 20 % |
| polyoxyethylene (2) stearyl ether [BRIJ® 72] | 6 % |
| polyoxyethylene (40) monostearate [MYRJ® 52] | 1 % |
| indomethacin | 1 % |
| water | 45 % |

The cream was prepared in the same manner as described in (f) above.

A 100 mg sample of the selected formulation was applied to the skin. Samples of reservoir fluid were assayed for indomethacin by HPLC using a μBondapak® RP-CN column with detection at 254 nm. The mobile phase was 2 mM $NH_4H_2PO_4$ in 40 % by volume acetonitrile/60 % by volume water.

### Table XX

| Indomethacin (1 %) in | 24 hour permeation percent |
|---|---|
| Petrolatum (a) | 0.9 |
| DEET (b) | 37.6 |
| 1 % DEET/petrolatum (c) | 1.1 |
| 5 % DEET/petrolatum (d) | 12.7 |
| 10 % DEET/petrolatum (e) | 21.8 |
| 5 % DEET oil/water cream (f) | 17.7 |
| 5 % DEET oil/water cream (g) | 14.6 |

### Example XXII

The in vitro diffusion cell procedure described previously was used to compare the permeation of the following formulations of tetracycline:

(a) 0.22 % tetracycline hydrochloride in petrolatum, prepared by suspending a mixture of tetracycline hydrochloride, 4-epicycline hydrochloride and sodium bisulfite in melted petrolatum, the product containing 2.2 mg of tetracycline hydrochloride per mL;

(b) 0.22 % tetracycline hydrochloride in 1 % DEET/petrolatum, prepared by warming a mixture of 1 g of DEET and 99 g of petrolatum until fluid (about 50°C), then adding a mixture of tetracycline hydrochloride, 4-epicycline hydrochloride and sodium bisulfite such that the product contains 2.2 mg of tetracycline hydrochloride per mL and mixing until cool;

(c) 0.22 % tetracycline hydrochloride in 5 % DEET/petrolatum, prepared as described in (b) above but using 5 g of DEET and 95 g of petrolatum;

(d) 0.44 % tetracycline hydrochloride in 1 % DEET/petrolatum, prepared by warming a mixture of 1 g of DEET and 99 g of petrolatum until fluid (about 50°C), then adding sufficient tetracycline hydrochloride to produce a product containing 4.4 mg of tetracycline hydrochloride per mL and mixing until cool;

(e) 0.44 % tetracycline hydrochloride in 5 % DEET/petrolatum, prepared as described in (d) above but using 5 g of DEET and 95 g of petrolatum;

(f) 0.44 % tetracycline hydrochloride in 10 % DEET/petrolatum, prepared as described in (d) above but using 10 g of DEET and 90 g of petrolatum.

A 100 mg sample of the selected formulation was applied to the skin. Samples of reservoir fluid were assayed for tetracycline by HPLC with detection at 254 nm. The mobile phase was 1 mM $Na_2EDTA$ and 2 mM $H_3PO$, in 28 % by volume acetonitrile/72 % by volume water.

### Table XXI

| Tetracycline | 24 hour permeation percent |
|---|---|
| 0.22 % in petrolatum (a) | 0 |
| 0.22 % in 1 % DEET/petrolatum (b) | 0.8 |
| 0.22 % in 5 % DEET/petrolatum (c) | 2.1 |
| 0.44 % in 1 % DEET/petrolatum (d) | 0.5 |
| 0.44 % in 5 % DEET/petrolatum (e) | 1.2 |
| 0.44 % in 10 % DEET/petrolatum (f) | 2.3 |

The various bio-affecting agents envisaged by the present invention are conveniently topically administered to warm-blooded animals in combination with DEET in conventional unit dosage amount and form. If desired, any suitable non-toxic or pharmaceutically acceptable topical carrier material or vehicle can be combined with DEET and the bio-affecting agent. Such carrier materials are per se well known to those skilled in the art of topical pharmaceutical formulations. Compare, for example, Remington's Pharmaceutical Sciences, 14th Edition (1970). Thus the formulation according to the invention may simply be a stable solution or suspension of the bio-affecting agent in DEET, or it may be a combination of the bio-affecting agent and DEET with one or more carrier materials to form a solution, suspension, lotion, cream, ointment or aerosol spray. Alternatively, a solution or suspension of the bio-affecting agent and DEET can be incorporated into a polymeric gel or film. The formulation of bio-affecting agent, DEET and, if desired, vehicle(s), can be applied directly to the skin, or can be applied to a carrier material such as a bandage, which can then be adhered to the skin. The concentration of DEET in the vehicle generally can vary from about 0.1 % to 100 %, while the concentration of the bio-affecting agent generally can vary from about 0.001 % to about 80 % of the total composition. The concentration of bio-affecting agent in the formulation will vary with many factors, e.g. its type and potency, the condition for which it is administered, the surface area to which it is applied, the type of formulation used and the concentration of DEET in the formulation. Therapeutic agents which are both dermally and systemically effective generally require the administration of considerably higher dosages to achieve a systemic effect than that needed to produce a dermal response; such higher dosages (which may be greater by a factor of 10 to 20 times in the case of an agent such as dexamethasone) can be achieved by increasing the concentration of DEET in the formulation, by increasing the concentration of the drug in the formulation, by increasing the area to which the formulation is applied, or by a combination of these measures.

**Claims** for the Contracting States: BE, CH, DE, FR, GB, IT, LI, LU, NL

1. A composition of matter for topical application comprising a biologically effective amount of at least one bio-affecting agent selected from antibacterial, antifungal, antiviral, corticosteroidal, steroidal antiinflammatory, non-steroidal antiinflammatory, antpsoriatic, antidermatitis, antipruritic, antiwart, keratolytic, local anesthetic, antierythema, antimetabolite, progestational, estrogenic, androgenic, emollient, skin lightening, antihistaminic, antiacne, antiperspirant, β-blocker, antihypertensive, antianginal, antiarrhythmic, vasodilator, antiemetic, muscle relaxant or antiasthma agents and a skin permeation enhancing amount of N,N-diethyl-m-toluamide with the exception of combinations of N,N-diethyl-m-toluamide with isopropanol or with shampoos, hair tonics or hair rinses.

2. A composition as defined by claim 1, further comprising a non-toxic topical carrier.

3. A composition as defined by claim 1 wherein said bio-affecting agent is lincomycin, clindamycin, tetracycline, oxytetracycline, chlorotetracycline, erythromycin, 2-thiopyridine N-oxide, iodine, an iodoantimicrobial, a penicillin antibiotic, a cephalosporin antibiotic, a sulfonamide antibacterial, hexachlorophene, chlorhexidine, a chloramine antibacterial, benzoylperoxide, an aminoglycoside antibiotic, nitrofurantoin, nystatin, amphotericin B, 5-iodo-2-deoxyuridine, griseofulvin, thiabendazole, gramicidin, 5-fluorouracil, 6-mercaptopurine, mycophenolic acid, methotrexate, indomethacin, naproxen, fenoprofen, ibuprofen, alcofenac, phenylbutazone, sulindac, desoxysulindac, diflunisal, aspirin, mefenamic acid, dibucaine, lidocaine, estradiol, testosterone, progesterone, cyproheptadiene hydrochloride, hydroquinone, methatropine nitrate, propantheline bromide, scopolamine, methscopolamine bromide, a quaternary acyloxymethylammonium salt, d,l-α-cyclopentylphenylacetoxymethyl-1-methylpyrrolidinium chloride, d,l-α-cyclopentylphenylacetoxymethyl-1-methylpyrrolidinium dodecyl sulfate, hydrocortisone, hydrocortisone 17-valerate, hydrocortisone 17-butyrate, hydrocortisone 21-acetate, betamethasone valerate, triamcinolone acetonide, fluocinonide, desonide, fluocinolone acetonide, dexamethasone, dexamethasone 21-phosphate, prednisolone, prednisolone 21-phosphate, haloprednone, N-formimidoyl thienamycin monohydrate, 1-ethyl-6-fluoro-1,4-dihydro-4-oxo-7-(1-piperazinyl)-3-quinoline carboxylic acid, phosphonomycin, novabiocin, cycloserine, cephamycin C, propranolol, bupranolol, metoprolol, nadolol, satalol, alprenolol, oxprenolol, carteolol, labetalol, atenolol, pindolol, timolol, timolol maleate, α-methyldopa, clonidine, nitroglycerin, erythritol tetranitrate, isosorbide dinitrate, mannitol hexanitrate, pentaerythrityl tetranitrate, papaverine, dipyridamole, cyclobenzaprine hydrochloride, diazepam, or cromoglycic acid.

**Claims** for the Contracting State: AT

1. A process for preparing a composition of matter for topical application comprising combining a biologically effective amount of at least one bio-affecting agent selected from antibacterial, antifungal, antiviral, corticosteroidal, steroidal antiinflammatory, non-steroidal antiinflammatory, antipsoriatic, antidermatitis, antipruritic, antiwart, keratolytic, local anesthetic, antierythema, antimetabolite, progestational, estrogenic, androgenic, emollient, skin lightening, antihistaminic, antiacne, antiperspirant, β-blocker, antihypertensive, antianginal, antiarrhythmic, vasodilator, antiemetic, muscle relaxant or antiasthma agents and a skin permeation enhancing amount of N,N-diethyl-m-toluamide with the exception of combining N,N-diethyl-m-toluamide with isopropanol or with shampoos, hair tonics or hair rinses.

2. A process as defined by claim 1, comprising additionally combining a non-toxic topical carrier.

3. A process as defined by claim 1 wherein said bio-affecting agent is lincomycin, clindamycin, tetracycline, oxytetracycline, chlorotetracycline, erythromycin, 2-thiopyridine N-oxide, iodine, an iodoantimicrobial, a penicillin antibiotic, a cephalosporin antibiotic, a sulfonamide antibacterial, hexachlorophene, chlorhexidine, a chloramine antibacterial, benzoylperoxide, an aminoglycoside antibiotic, nitrofurantoin, nystatin, amphotericin B, 5-iodo-2-deoxyuridine, griseofulvin, thiabendazole, gramicidin, 5-fluorouracil, 6-mercaptopurine, mycophenolic acid, methotrexate, indomethacin, naproxen, fenoprofen, ibuprofen, alcofenac, phenylbutazone, sulindac, desoxysulindac, diflunisal, aspirin, mefenamic acid, dibucaine, lidocaine, estradiol, testosterone, progesterone, cyproheptadiene hydrochloride, hydroquinone, methatropine nitrate, propantheline bromide, scopolamine, methscopolamine bromide, a quaternary acyloxymethylammonium salt, d,l-α-cyclopentyl-phenylacetoxymethyl-1-methylpyrrolidinium chloride, d,l-α-cyclopentylphenylacetoxymethyl-1-methylpyrrol-idinium dodecyl sulfate, hydrocortisone, hydrocortisone 17-valerate, hydrocortisone 17-butyrate, hydrocortisone 21-acetate, betamethasone valerate, triamcinolone acetonide, fluocinonide, desonide, fluocinolone acetonide, dexamethasone, dexamethasone 21-phosphate, prednisolone, prednisolone 21-phosphate, haloprednone, N-formimidoyl thienamycin monohydrate, 1-ethyl-6-fluoro-1,4-dihydro-4-oxo-7-(1-piperazinyl)-3-quinoline carboxylic acid, phosphonomycin, novabiocin, cycloserine, cephamycin C, propranolol, bupranolol, metoprolol, nadolol, satalol, alprenolol, oxprenolol, carteolol, labetalol, atenolol, pindolol, timolol, timolol maleate, α-methyldopa, clonidine, nitroglycerin, erythritol tetranitrate, isosorbide dinitrate, mannitol hexanitrate, pentaerythrityl tetranitrate, papaverine, dipyridamole, cyclobenzaprine hydrochloride, diazepam, or cromoglycic acid.

**Patentansprüche** für die Vertragstaaten: BE, CH, DE, FR, GB, IT, LI, LU, NL

1. Eine Zusammensetzung zur topischen Anwendung, enthaltend eine biologisch wirksame Menge wenigstens eines biologisch beeinflussenden Mittels, ausgewählt aus antibakteriellen, Antifungus-, antiviralen, Corticosteroid-, entzündungshemmenden Steroid-, entzündungshemmenden Nichtsteroid-, Antipsoriasis-, Antidermatitis-, Antipruritis-, Antiwarzen-, keratolytischen, lokalanästhetischen, Antierythema-, Antimetabolit-, progestativen, östrogenen, androgenen, Erweichungs-, Hautaufhellungs-, Antihistamin-, Antiakne- , Antitranspirans-, β-Blocker-, antihypertensiven, antianginalen, antiarrhythmischen, Vasodilator-, antiemetischen, muskelrelaxierenden oder Antiasthma-Mitteln,und eine die Hautdurchdringung erhöhende Menge von N,N-Diethylm-toluamid, mit der Ausnahme von Kombinationen von N,N-Diethyl-m-toluamid mit Isopropanol oder mit Shampoos, Haartonika oder Haarspülmitteln.

2. Eine Zusammensetzung wie in Anspruch 1 definiert, enthaltend außerdem einen nichttoxischen, topischen Träger.

3. Eine Zusammensetzung wie in Anspruch 1 definiert, worin das biologisch beeinflussende Mittel Lincomycin, Clindamycin, Tetracyclin, Oxytetracyclin, Chlortetracyclin, Erythromycin, 2-Thiopyridin-N-oxid, Iod, ein antimikrobielles Mittel auf Iodbasis, ein Penicillinantibiotikum, ein Cephalosporinantibiotikum, ein antibakterielles Mittel auf Sulfonamidbasis, Hexachlorophen, Chlorhexidin, ein antibakterielles Mittel auf Chloraminbasis, Benzoylperoxid, ein Aminoglykosid-Atibiotikum, Nitrofurantoin, Nystatin, Amphotericin B, 5-Iod-2-deoxyuridin, Griseofulvin, Thiabenzadol, Gramicidin, 5-Fluoruracil, 6-Mercaptopurin, Mycophenolsäure, Methotrexat, Indomethacin, Naproxen, Fenoprofen, Ibuprofen, Alcofenac, Phenylbutazon, Sulindac, Desoxysulindac, Diflunisal, Aspirin, Mefenaminsäure, Dibucain, Lidocain, Östradiol, Testosteron, Progesteron, Cyproheptadienhydrochlorid, Hydrochinon, Methatropinnitrat, Propanthelinbromid, Scopolamin, Methscopolaminbromid, ein quaternäres Acyloxymethylammoniumsalz, d,l-α-Cyclopentylphenylacetoxymethyl-1-methylpyrrolidiniumchlorid, d,l-α-Cyclopentylphenylacetoxymethyl-1-methylpyrrolidiniumdodecylsulfat, Hydrocortison, Hydrocortison-17-valerat, Hydrocortison-17-butyrat, Hydrocortison-21-acetat, Betamethasonvalerat, Triamcinolonacetonid, Fluocinonid, Desonid, Fluocinolonacetonid, Dexamethason, Dexamethason-21-phosphat, Prednisolon, Prednisolon-21-phosphat, Halogenprednon, N-Formimidoylthienamycinmonohydrat, 1-Ethyl-6-fluor-1,4-dihydro-4-oxo-7-(1-piperazinyl)-3-chinolincarbonsäure, Phosphonomycin, Novabiocin, Cycloserin, Cephamycin C, Propranolol, Bupranolol, Metoprolol, Nadolol, Satalol, Alprenolol, Oxprenolol, Carteolol, Labetalol, Atenolol, Pindolol, Timolol, Timololmaleat, α-Methyldopa, Clonidin, Nitroglycerin, Erythrittetranitrat, Isosorbiddinitrat, Mannithexanitrat, Pentaerythrityltetranitrat, Papaverin, Dipyridamol, Cyclobenzaprinhydrochlorid, Diazepam oder Cromoglycinsäure ist.

**Patentansprüche** für den Vertragstaat: AT

1. Ein Verfahren zur Herstellung einer Zusammensetzung zur topischen Anwendung, umfassend das Vereinigen einer biologisch wirksamen Menge wenigstens eines biologisch beeinflussenden Mittels, ausgewählt aus antibakteriellen, Antifungus-, antiviralen, Corticosteroid-, entzündungshemmenden Steroid-, entzündungshemmenden Nichtsteroid-, Antipsoriasis-, Antidermatitis-, Antipruritis-, Antiwarzen-, keratolytischen, lokalanästhetischen, Antierythema-, Antimetabolit-, progestativen, östrogenen, androgenen, Erweichungs-, Hautaufhellungs-, Antihistamin-, Antiakne-, Antitranspirans-, β-Blocker-, antihypertensiven, antianginalen, antiarrhythmischen, Vasodilator-, antiemetischen, muskelrelaxierenden oder Antiasthma-Mitteln, und einer die Hautdurchdringung erhöhenden Menge von N,N-Diethyl-m-toluamid, ausgenommen das Vereinigen von N,N-Diethyl-m-toluamid mit Isopropanol oder mit Shampoos, Haartonika oder Haarspülmitteln.

2. Ein Verfahren wie in Anspruch 1 definiert, umfassend zusätzlich das Vereinigen mit einem nichttoxischen, topischen Träger.

3. Ein Verfahren wie in Anspruch 1 definiert, worin das biologisch beeinflussende Mittel Lincomycin, Clindamycin, Tetracyclin, Oxytetracyclin, Chlortetracyclin, Erythromycin, 2-Thiopyridin-N-oxid, Iod, ein antimikrobielles Mittel auf Iodbasis, ein Penicillinantibiotikum, ein Cephalosporinantibiotikum, ein antibakterielles Mittel auf Sulfonamidbasis, Hexachlorophen, Chlorhexidin, ein antibakterielles Mittel auf Chloraminbasis, Benzoylperoxid, ein Aminoglykosid Antibiotikum, Nitrofurantoin, Nystatin, Amphotericin B, 5-Iod-2-deoxyuridin, Griseofulvin, Thiabenzadol, Gramicidin, 5-Fluoruracil, 6-Mercaptopurin, Mycophenolsäure, Methotrexat, Indomethacin, Naproxen, Fenoprofen, Ibuprofen, Alcofenac, Phenylbutazon, Sulindac, Desoxysulindac, Diflunisal, Aspirin, Mefenaminsäure, Dibucain, Lidocain, Östradiol, Testosteron, Trogesteron, Cyproheptadienhydrochlorid, Hydrochinon, Methatropinnitrat, Propanthelinbromid, Scopolamin, Methscopolaminbromid, ein quaternäres Acyloxymethylammoniumsalz, d,l-α-Cyclopentylphenylacetoxymethyl-1-methylpyrrolidiniumchlorid, d,l-α-Cyclopentylphenylacetoxymethyl-1-methylpyrrolidiniumdodecylsulfat, Hydrocortison, Hydrocortison-17-valerat, Hydrocortison-17-butyrat, Hydrocortison-21-acetat, Betamethasonvalerat, Triamcinolonacetonid, Fluocinonid, Desonid, Fluocinolonacetonid, Dexamethason, Dexamethason-21-phosphat, Prednisolon, Prednisolon-21-phosphat, Halogenprednon, N-Formimidoylthienamycinmonohydrat, 1-Ethyl-6-fluor-1,4-dihydro-4-oxo-7-(1-piperazinyl)-3-chinolincarbonsäure, Phosphonomycin, Novabiocin, Cycloserin, Cephamycin C, Propranolol, Bupranolol, Metoprolol, Nadolol, Satalol, Alprenolol, Oxprenolol, Carteolol, Labetalol, Atenolol, Pindolol, Timolol, Timololmaleat, α-Methyldopa, Clonidin, Nitroglycerin, Erythrittetranitrat, Isosorbiddinitrat, Mannithexanitrat, Pentaerythrityltetranitrat, Papaverin, Dipyridamol, Cyclobenzaprinhydrochlorid, Diazepam oder Cromoglycinsäure ist.


**Revendications** pour les Etats Contractants: BE, CH, DE, FR, GB, IT, LI, LU, NL

1. Une composition pour l'application locale comprenant une quantité efficace biologiquement d'au moins un agent bio-actif choisi parmi les agents antibactériens, antifongiques, antiviraux, corticostéroïdes, antiinflammatoires stéroïdiens, antiinflammatoires non stéroïdiens, antipsoriasiques, antidermatites, antipruritiques, antiverrues, kératolytiques, anesthésiques locaux, antiérythèmes, antimétabolites, progestatifs, oestrogènes, androgènes, émollients écrans solaires, éclaircissants de la peau, antihistaminiques, antiacné, antisudoraux, β-bloquants, antihypertenseurs, antiangineux, antiarythmiques, vasodilatateurs, antiémétiques, myorelaxants ou antiasthmatiques et d'une quantité accroissant la pénétration dans la peau de N,N-diéthyl-m-toluamide excepté les combinaisons de N,N-diéthyl-m-toluamide, avec de l'isopropanol ou avec des shampoings, lotions de rinçage ou tonifiante pour les cheveux.

2. Une composition comme définie par la revendication 1 comprenant de plus la combinaison d'un support à usage local non toxique.

3. Une composition comme définie par la revendication 1 dans laquelle ledit agent bio-actif est la lincomycine, la clindamycine, la tétracycline, l'oxytétracycline, la chlorotétracycline, l'érythromycine, le 2-thiopyridine N-oxyde, l'iode, un agent antibactérien iodé, un antibiotique du groupe de la pénicilline, un antibiotique du groupe de la céphalosporine, un antibactérien du groupe des sulfamides, l'hexachlorophène, la chlorhexidine, un antibactérien du groupe de la chloramine, le peroxyde de benzoyle, un antibiotique aminoglucosidique, la nitrofurantoïne, la nystatine, l'amphotéricine B, la 5-iodo-2-désoxyuridine, la griséofulvine, le thiabendazole, la gramicidine, le 5-fluorouracile, la 6-mercaptopurine, l'acide mycophénolique, le méthotrexate, l'indométhacine, le napro naproxen, le fénoprofen, l'ibuprofen, l'alcofenac, la phénylbutazone, le sulindac, le désoxysulindac, le diflunisal, l'aspirine, l'acide méfénamique, la benzocaïne, la procaïne, la propoxycaïne, la dibucaïne, la lidocaïne, l'acide p-aminobenzoïque, l'acide pdiméthylaminobenzoïque, un ester alkylique de l'acide p-diméthylaminobenzoïque, l'estradiol, la testostérone, la progestérone, le chlorhydrate de cyproheptadine, l'hydroquinone, le nitrate de méthatropine, le bromure de propanthéline, la scopolamine, le bromure de méthaméthscopolamine, un sel quaternaire d'acyloxyméthylammonium, le chlorure de d,l-a-cyclopentylphénylacétoxy-méthyl-1-méthylpyrrolidinium, le dodécylsulfate de d,l-α-cyclopentylphénylacétoxyméthyl-1-méthylpyrrolidinium, l'hydrocortisone, le 17-valérate d'hydrocortisone, le 17-butyrate d'hydrocortisone, le 21-acétate d'hydrocortisone, le valérate de β-méthasone, l'acétonide de triamcinolone, le fluocinonide, le désonide, l'acétonide de fluocinolone, la dexaméthasone, le 21-phosphate de dexaméthasone, la prednisolone, le 21-phosphate de prednisolone, l'haloprednone, la N-formimidoyl thiénamycine monohydratée, l'acide 1-éthyl-6-fluoro-1,4-dihydro-4-oxo-7-(1-pipérazinyl)-3-quinoléine carboxylique, la phosphonomycine, la novobiocine, la cyclosérine, la céphamycine C, le propanolol, le bupranolol, le métoprolol, le nadolol, le satalol, l'alprénolol, l'oxprénolol, le cartéolol, le labétalol, l'aténolol, le pindolol, le timolol, le maléate de timolol, l'α-méthyldopa, la clonidine, la nitroglycérine, le tétranitrate d'érythritol, le dinitrate d'isosorbide, l'hexanitrate de mannitol, le tétranitrate de pentaérythrityle, la papavérine, le dipyridamol, le chlorhydrate de cyclobenzaprine, le diazépam ou l'acide cromoglycique.

**Revendications** pour l'Etat Contractant: AT

1. Un procédé de préparation d'une composition pour l'application locale comprenant la combinaison d'une quantité efficace biologiquement d'au moins un agent bio-actif choisi parmi les agents antibactériens, antifongiques, antiviraux, corticostéroïdes, antiinflammatoires stéroïdiens, antiinflammatoires non stéroïdiens, antipsoriasiques, antidermatites, antipruritiques, antiverrues, kératolytiques, anesthésiques locaux, antiérythèmes, antimétabolites, progestatifs, oestrogènes, androgènes, émollients écrans solaires, éclaircissants de la peau, antihistaminiques, antiacné, antisudoraux, β-bloquants, antihypertenseurs, antiangineux, antiarythmiques, vasodilatateurs, antiémétiques, myorelaxants ou antiasthmatiques et d'une quantité accroissant la pénétration dans la peau de N,N-diéthyl-m-toluamide excepté les combinaisons de N,N-diéthyl-m-toluamide, avec de l'isopropanol ou avec des shampoings, lotions de rinçage ou tonifiantes pour les cheveux.

2. Un procédé comme défini dans la revendication 1 comprenant de plus la combinaison d'un support à usage local non toxique.

3. Un procédé comme défini dans la revendication 1 dans lequel ledit agent bio-actif est la lincomycine, la clindamycine, la tétracycline, l'oxytétracycline, la chlorotétracycline, l'érythromycine, le 2-thiopyridine N-oxyde, l'iode, un agent antibactérien iodé, un antibiotique du groupe de la pénicilline, un antibiotique du groupe de la céphalosporine, un antibactérien du groupe des sulfamides, l'hexachlorophène, la chlorhexidine, un antibactérien du groupe de la chloramine, le peroxyde de benzoyle, un antibiotique aminoglucosidique, la nitrofurantoïne, la nystatine, l'amphotéricine B, la 5-iodo-2-désoxyuridine, la griséofulvine, le thiabendazole, la gramicidine, le 5-fluorouracile, la 6-mercaptopurine, l'acide mycophénolique, le méthotrexate, l'indométhacine, le napro naproxen, le fénoprofen, l'ibuprofen, l'alcofenac, la phénylbutazone, le sulindac, le désoxysulindac, le diflunisal, l'aspirine, l'acide méfénamique, la benzocaïne, la procaïne, la propoxycaïne, la dibucaïne, la lidocaïne, l'acide p-aminobenzoïque, l'acide p-diméthylaminobenzoïque, un ester alkylique de l'acide p-diméthylaminobenzoïque, l'estradiol, la testostérone, la progestérone, le chlorhydrate de cyproheptadine, l'hydroquinone, le nitrate de méthatropine, le bromure de propanthéline, la scopolamine, le bromure de méthaméthscopolamine, un sel quaternaire d'acyloxyméthylammonium, le chlorure de d,l-α-cyclopentylphé-nylacétoxyméthyl-1-méthylpyrrolidinium, le dodécylsulfate de d,l-α-cyclopentylphénylacétoxyméthyl-1-méthyl-pyrrolidinium, l'hydrocortisone, le 17-valérate d'hydrocortisone, le 17-butyrate d'hydrocortisone, le 21-acétate d'hydrocortisone, le valérate de β-méthasone, l'acétonide de triamcinolone, le fluocinonide, le désonide, l'acétonide de fluocinolone, la dexaméthasone, le 21-phosphate de dexaméthasone, la prednisolone, le 21-phosphate de prednisolone, l'haloprednone, la N-formimidoyl thiénamycine monohydratée, l'acide 1-éthyl-6-fluoro-1,4-dihydro-4-oxo-7-(1-pipérazinyl)-3-quinoléine carboxylique, la phosphonomycine, la novobiocine, la cyclosérine, la céphamycine C, le propanolol, le bupranolol, le métoprolol, le nadolol, le satalol, l'alprénolol, l'oxprénolol, le cartéolol, le labétalol, l'aténolol, le pindolol, le timolol, le maléate de timolol, l'α-méthyldopa, la clonidine, la nitroglycérine, le tétranitrate d'érythritol, le dinitrate d'isosorbide, l'hexanitrate de mannitol, le tétranitrate de pentaérythrityle, la papavérine, le dipyridamol, le chlorhydrate de cyclobenzaprine, le diazépam ou l'acide cromoglycique.